# EUROPEAN PATENT APPLICATION

(11) **EP 1 681 289 A1**
(43) Date of publication of application: **19.07.2006**
(21) Application number: 04817426.2
(22) Date of filing: 04.11.2004
(51) Int. Cl.: C07D 311/30, A61K 31/352, A61K 31/7048, A61K 36/18, A61P 3/04, A61P 3/10, A61P 43/00, A23L 1/30

(54) **PLANT-ORIGIN BETA3-ADRENOCEPTOR AGONIST AND USE OF THE SAME**

(30) Priority: 04.11.2003 JP 2003374836
(71) Applicant: Meiji Dairies Corporation, Tokyo 136-8908 (JP)
(72) Inventor: TSUBOI, Hiroshi, c/o MEIJI DAIRIES CORPORATION, Kanagawa 250-0862 (JP); IKEGAMI, Shuji, c/o MEIJI DAIRIES CORPORATION, Kanagawa 250-0862 (JP); KAMIYAMA, Tomonori, c/o MEIJI DAIRIES CORPORATION, Kanagawa 250-0862 (JP); JI, Zai-si, c/o MEIJI DAIRIES CORPORATION, Kanagawa 250-0862 (JP); ASAMI, Yukio, c/o MEIJI DAIRIES CORPORATION, Kanagawa 250-0862 (JP); ITOU, Hiroyuki, c/o MEIJI DAIRIES CORPORATION, Kanagawa 250-0862 (JP); ODA, Munehiro, c/o MEIJI DAIRIES CORPORATION, Kanagawa 250-0862 (JP); SHIN, Kazuo, c/o MEIJI DAIRIES CORPORATION, Kanagawa 250-0862 (JP)
(74) Representative: Grünecker, Kinkeldey, Stockmair & Schwanhäusser Anwaltssozietät
(86) International application number: PCT/JP2004/016330
(87) International publication number: WO 2005/042508

(57) **Abstract**

The present inventors produced lotus leaf extracts, and discovered that quercetin is one of the active ingredients. As a result of treating β₃-adrenergic receptor-expressing cells and feeding diabetes model mice with quercetin and evaluating its effects, the present inventors discovered specifically that quercetin produces obesity-improving effects and antidiabetic effects by acting as a β₃-adrenergic receptor agonist.

## Description

### Technical Field

The present invention relates to novel β₃-adrenergic receptor agonist substances prepared from lotus leaves.

### Background Art

The ratio of obese individuals is increasing worldwide as lifestyles become modernized and Westernized. Since obesity leads to lifestyle diseases such as diabetes, high blood pressure and arteriosclerosis, and increases the mortality rate, prevention and treatment of obesity is a critical public health issue. The basics of obesity treatment are diet therapy and exercise therapy; however, drug therapy may also be introduced for cases where improvement is difficult by such therapies.

Obesity is a condition characterized by excessive accumulation of neutral fat (triglyceride) in adipocytes. There are white fat and brown fat in adipocytes. White adipocytes are relatively large cells that are widely distributed throughout the body, for example, below the skin and around the intestines; and most of the cell body is occupied by enormous lipid droplets. On the other hand, brown adipocytes are localized at the interscapulum, subaxillary region and such, and the fat is separated into small droplets, forming a multilocular structure with many mitochondria nearby. The physiological functions of the white fat and brown fat differ greatly. White fat is where excessive energy is stored, whereas brown fat is where energy is released as heat through oxidative degradation of fat. Fat stored in white adipocytes is degraded into fatty acids under conditions of energy shortage, and released into the blood to be consumed by the whole body; whereas brown fat is degraded into fatty acids through stimulation, and immediately oxidized in brown adipocytes to generate heat (Non-Patent Document 1: Saito M., Sasaki N. Jikken igaku (Experimental Medicine) Vol. 14, No. 16, 1996).

β₃-adrenergic receptors are known to be involved in lipolysis. β-adrenergic receptors can be classified into subtypes β₁, β₂, and β₃. All of them are seven-transmembrane receptors comprising approximately 400 amino acids, although the amino acid homology between β₁ and β₂ is only about 50%. β₁ receptors are present mainly in the heart and such, and β₂ receptors are present mainly in the bronchial smooth muscles and such, while β₃ receptors are present mainly in the adipose tissues and tissues such as the intestinal tract and brain.

β3-adrenergic receptor agonists (agonist substances) are known to promote the accumulation of cAMP in adipocytes (Non-Patent Document 2: Igaku no Ayumi (Progress in Medicine) Vol. 192, No.5, 2001.1.29). When β₃-adrenergic receptor agonists promote lipolysis in white adipocytes, at the same time, they activate brown adipocytes. Activation of β₃-adrenergic receptors is known to achieve effects such as increased thermogenesis; activated brown fat; mitigated obesity such as decrease in body fat; and reduced insulin resistance (Non-Patent Document 3: J. Clin. Invest. 1996 Jun 15, 97(12):2898-904; Life Sci.1994, 54(7):491-8). So far, several β₃-adrenergic receptor agonists have been developed as antiobesity agents and antidiabetic agents, for example, "AJ-9677" of Dainippon

### Pharmaceuticals and Takeda Pharmaceuticals.

Besides the food application of the roots of *Nelumbo nucifera,* a perennial lotus plant of the *Nelumbonaceae,* its seeds and leaves are widely used in Chinese herbal medicine formulations and health foods. Lotus leaves are known to have an obesity-improving effect (Patent Document 1: Japanese Patent Application Kokai Publication No. (JP-A) H8-198769); however, there are no detailed reports on the active ingredients derived from lotuses which exhibit such effects on obesity, or their functions.
[Patent Document 1] JP-A H8-198769
[Non-Patent Document 1] Saito M., Sasaki N. Jikken igaku (Experimental Medicine) Vol. 14, No. 16, 1996
[Non-Patent Document 2] Igaku no Ayumi (Progress in Medicine) Vol. 192, No.5, 2001.1.29
[Non-Patent Document 3] J. Clin. Invest. 1996 Jun 15, 97(12):2898-904; Life Sci.1994, 54(7):491-8
[Non-Patent Document 4] Biochemical Pharmacology, Vol.47, No.3, pp521-529

### Disclosure of the Invention

### Problems to be Solved by the Invention

The present invention was made in view of the above circumstances. An objective of the present invention is to first identify an active ingredient in *Nelumbonaceae* plants, and then provide novel substances that can be produced based on the active ingredient, or more specifically, β₃-adrenergic receptor agonistic substances.

### Means to Solve the Problems

To solve the above-mentioned objective, the present inventors dedicated themselves to produce lotus leaf extracts and identify active ingredients with obesity-improving effects. As a result of their dedicated research, the present inventors discovered one such active ingredient - quercetin. Although quercetin is a type of flavonoid that exists widely in plants, it is the present inventors who discovered for the first time that lotus contains quercetin. For the quercetin-related findings, so far there is one report that suggests quercetin has rat β-adrenergic receptor agonist activity based on the fact that cAMP accumulates as a result of treating rat adipocytes with quercetin (Biochemical Pharmacology, Vol.47, No.3, pp521-529). However, agonistic activity towards human β₃-adrenergic receptor (β3AR) has not been confirmed. The present inventors treated β₃-adrenaline receptor-expressing cells and diabetes model mice with quercetin. From evaluating the effects, the present inventors discovered specifically that obesity-improving effects and antidiabetic effects are achieved as a result of quercetin functioning as a β₃-adrenergic receptor agonist. Furthermore, the present inventors administered a quercetin-containing lotus leaf extract to human patients with borderline diabetes and confirmed the body-fat-reducing effect in human indeed. Specifically, the present inventors discovered that pharmaceuticals and foods with effects of improving obesity and diabetes can be developed by blending lotus leaf preparations, and thereby completed the present invention.

That is, the present invention relates to novel β₃-adrenergic receptor agonistic substances prepared from lotus leaves, and specifically relates to the following invention:
(1) a β₃-adrenergic receptor agonist substance comprising quercetin;
(2) the substance of (1), wherein the quercetin is derived from a plant;
(3) the substance of (2), wherein the plant is a *Nelumbonaceae* plant;
(4) a pharmaceutical agent for treating or preventing diabetes, wherein the agent comprises the substance of any one of (1) to (3);
(5) a pharmaceutical agent for treating or preventing obesity, wherein the agent comprises the substance of any one of (1) to (3), and has an effect of improving lipid metabolism;
(6) a food for treating or preventing diabetes, wherein the food comprises the substance of any one of (1) to (3);
(7) a food for treating or preventing obesity, wherein the food comprises the substance of any one of (1) to (3); and
(8) a β₃-adrenergic receptor agonist substance which comprises a lotus preparation comprising quercetin.

### Brief Description of the Drawings

Fig. 1 shows the accumulation of cAMP in CHO-K1 cells as a result of adding a lotus leaf extract. Mean ± S.D. (n=3)
Fig. 2 shows the accumulation of cAMP in CHO-K1 cells caused by quercetin. Mean ± S.D. (n=3)
Fig. 3 shows the accumulation of cAMP in CHO-K1 cells caused by quercetin and Q3GA (Quercetin 3-O-β-D-glucuronide). Mean ± S.D. (n=3)
Fig. 4 shows the amount of glycerol released from 3T3-L1 cells due to the lotus leaf extract and quercetin. Mean ± S.D. (n=3)
Fig. 5 shows the hypoglycemic effect observed when the lotus leaf extract was administered to type-II diabetes mouse models. Mean ± S.D. (n=10)
Fig. 6 shows the blood glucose level in type-II diabetes mouse models on day 25 of the lotus leaf extract administration. Mean ± S.D. (n=10)
Fig. 7 shows the glucose tolerance test results obtained from the lotus leaf extract-administered group (humans) and the control group (humans). Blood glucose levels at certain times after glucose loading are shown as relative glucose levels (%) with respect to the glucose level before glucose loading. Mean ± S.D. Group S (placebo), n=30; Group T (dried lotus leaf extract 1 g/day), n=34; and Group R (dried lotus leaf extract 2 g/day), n=31.

### Best Mode for Carrying Out the Invention

The present invention provides quercetin-containing β₃-adrenergic receptor agonistic substances. As described above, the present invention is based on the finding that a lotus leaf extract contains quercetin as an active ingredient, and that this quercetin has β₃-adrenergic receptor agonist activity.

Quercetin is formally called 3,3',4',5,7-pentahydroxyflavone with an assigned CAS NO. of 117-39-5, and it is a type of flavonol that is widely distributed in plants. Its properties include yellow fine needle-like crystal, 316-317°C melting point, and two molecules of water of crystallization. It is insoluble in cold water, slightly soluble in boiling water, readily soluble in hot alcohol-glacial acetic acid, and poorly soluble in cold alcohol-ether. Since various sugars bind to position 3 or position 7, or both positions of quercetin, many quercetin glycosides are found, and quercetin mainly exists as a glycoside in plants (Seikagaku-jiten (Dictionary of Biochemistry) (3rd edition), Tokyo Kagaku Dojin; Seikagaku-daijiten (Comprehensive Dictionary of Chemistry), Tokyo Kagaku Dojin). Generally, quercetin thus refers to a non-glycosylated (aglycone) state, but "quercetin" as used herein is not limited to the above-mentioned non-glycosylated form (3,3',4',5,7-pentahydroxyflavone), and may include quercetin glycosides, as long as it functions as a β₃-adrenergic receptor agonist substance. Examples of the glycosides include quercetin-3-glucuronide, quercetin-3-glucoside (isoquercitrin), quercitrin, quercimeritrin, and rutin. Among them, quercetin-3-glucuronide, which is also referred to as quercetin 3-O-β-D-glucuronide or Q3GA, has a molecular weight of 478 and a melting point of 182-195°C, and is the assigned CAS NO. 22688-79-5 substance. The present inventors indeed confirmed that Q3GA, which is a quercetin glycoside, has β₃-adrenergic receptor agonist activity. Whether such a glycoside has β₃-adrenergic receptor agonist activity can be confirmed based on the enhancement of intracellular cAMP accumulation, which takes place when a β₃-adrenergic receptor is stimulated. More specifically, the activity as a β₃-adrenergic receptor agonist substance can be confirmed by adding a test substance to a β₃-adrenergic receptor-expressing cell, and measuring the accumulation of cAMP as described in the Examples. cAMP activity can be measured by immunoassays well known to those skilled in the art, such as EIA, ELISA, and RIA. A commercially available kit may also be used.

The quercetin of the present invention can be made into a quercetin-comprising β₃-adrenergic receptor agonist substance without limitation to the species or section of plant body, as long as it is a quercetin. In the present invention, the substance was found in a lotus leaf extract, but as long as there is β₃-adrenergic receptor agonist activity, quercetin prepared from plants other than lotus may be included. Examples of plants that have been known to contain quercetin include onion, broccoli, tea, ginko leaves, spinach, kale, parsley, celery, brussels sprouts, asparagus, apples, pears, guava leaves, beans, bell peppers, Jew's mallow, oranges, and strawberries. It is also thought to exist in *Nelumbonaceae* plants to which lotus belongs, and in closely related *Nymphaea, Euryale, Nuphar,* and *Brasenia.* Furthermore, rutin is known to exist in buckwheat and tomato. Therefore, the quercetin to be used as a β₃-adrenergic receptor agonist substance can be obtained from these plants. Quercetin can be prepared from plants, for example, by extracting quercetin from lotus leaves as described later in the Examples.

Representative examples of the section of a plant body to used for obtaining quercetin is the leaf, but other section such as flower, root, stem, fruit, pericarp, bark, rhizome, tuber, seeds, stigma, sap, and essential oils may be used as long as the section contains quercetin. Different sections may be used depending on the plant.

The above-mentioned quercetin can be effectively purified from plants by methods well known to those skilled in the art. For example, a plant is used as it is, or after being subjected to a grinding or fragmentation process. This plant (or processed plant) is then extracted by adding a solvent such as water or ethanol, and quercetin can be purified from the extract by techniques commonly known to those skilled in the art such as liquid chromatography based on the known properties of quercetin. An example of quercetin isolation from plants is isolation from *Rhododendron cinnabarinum* Hook, *Ericaceae* reported in Rangaswami *et al.,* Proc. Indian Acad. Sci. 56A, 239 (1962). An example of Q3GA isolation from beans has been reported in Price KR *et al.,* J. Agric. Food Chem., 46(12), 4898-4903 (1998). In addition to products purified from plants, chemically synthesized products, products purified from natural sources other than plants, those that are biologically synthesized using microorganisms, and such may also be used as a quercetin-comprising β₃-adrenergic receptor agonist substance of the present invention. Besides plants, quercetin is known to be present in propolis and red wine. Known examples of biosynthesis include those described in Watkin *et al.,* ibid. 229; Grisebach, Biochem. J. 85, 3p (1962); Patschke *et al.,* Z. Naturforsch. 21b, 201 (1966); Synthesis: Shakhova *et al.,* Zh.Obshch. Khim. 32, 390 (1962); and C.A.58, 1426f (1963). Examples of reports on Q3GA synthesis include Tetrahedron Letters, 43(35), 6263-6266 (2002); and Moon, Jae-Haka *et al.,* Free Radical Biology & Medicine, 30(11), 1274-1285 (2001).

The above-mentioned β₃-adrenergic receptor agonist activity of quercetin has been confirmed in the human and mouse receptors; therefore, quercetin can be used as an agonist (agonist substance) of mammalian β₃-adrenergic receptors such as those of human and rodents including mouse. There are reports that β₃-adrenergic receptor agonists have effects of activating brown adipocytes while promoting lipolysis in white adipocytes, increasing thermogenesis, activating brown fat, improving obesity by reducing body fat, reducing insulin resistance, and such. Therefore, the quercetin of the present invention may be used particularly as a pharmaceutical agent for treating or preventing obesity, or a pharmaceutical agent for treating or preventing diabetes. In addition, the β₃-adrenergic receptor agonist activity may also be used to treating diseases. When using quercetin as the above-mentioned β₃-adrenergic receptor agonist, quercetin collected from the above-mentioned plants can be used as it is, or after being modified following its collection from plants as long as the β₃-adrenergic receptor agonist activity is not impaired, or if the activity can be used more efficiently. Purification of quercetin is not absolutely required, and as long as quercetin is included, extracts, dried plant extracts, or plant preparations such as powdered plants can be used as a quercetin-comprising β₃-adrenergic receptor agonist substance.

The present invention provides pharmaceutical agents and foods for treating or preventing diabetes, which comprise the quercetin-comprising human β₃-adrenergic receptor agonist substances of the present invention. Diabetes characterized by constant high blood glucose due to insufficient insulin action is a disease group whose development relates to genetic and environmental factors. There is not a single cause or pathology for diabetes and it is classified according to the cause of onset or the degree of insulin action insufficiency. Based on the cause of onset, diabetes can be classified into type 1 diabetes, type 2 diabetes, other specific types, and gestational diabetes. A characteristic of the onset of type 1 diabetes is the destruction of the β cells in the pancreatic islets of Langerhans. For type 2 diabetes, decrease of insulin secretion and decrease of insulin sensitivity (insulin resistance) are both involved in its onset. Diabetes that accompanies other diseases is classified as other specific types. Based on the degree of insulin action insufficiency, diabetes can be classified into conditions that are insulin dependent or insulin independent. Type 1 diabetes is mostly an insulin dependent condition. Type 2 diabetes is mostly an insulin independent condition, and can be further classified into conditions that require insulin treatment and those that do not require insulin treatment for improving high blood glucose. As described above, activation of β₃-adrenergic receptor is known to result in an insulin resistance-reducing effect; therefore, pharmaceutical agents that comprise quercetin, a β₃-adrenergic receptor agonist substance, can be used for treating or preventing diabetes. The pharmaceutical agents of the present invention are effective against diabetes such as type 2 diabetes, but also encompass agents that are effective against other types of diabetes, as long as the pharmaceutical agents comprise a quercetin-comprising β₃-adrenergic receptor agonist substance and are intended for treating or preventing diabetes.

Whether or not a pharmaceutical agent or food is effective against diabetes can be determined, for example, by administering the pharmaceutical agent to a test animal that has developed diabetes, and measuring the blood glucose level of the test animal, as described in the Examples of the present invention. A comparison of the blood glucose levels before and after administration of a pharmaceutical agent, or the fasting blood glucose level is made between a control group and a group to which the pharmaceutical agent has been administered. The pharmaceutical agent is determined to be effective against diabetes if the blood glucose level of the pharmaceutical agent-administered group is lower than that of the control group. For the test animal, model animals with natural onset of type II diabetes (for example, KK-A^{y} mice) and model animals with high fat diet-induced obesity can be used. Model animals that have artificially developed diabetes as a result of streptozotocin administration, and model animals with natural onset of type I diabetes may also be used.

The present invention also provides pharmaceutical agents and foods for treating or preventing obesity, which have effects of improving lipid metabolism and comprise a quercetin-comprising β₃-adrenergic receptor agonist substance. As described above, β₃-adrenergic receptor agonist substances have lipid metabolizing effects; therefore, quercetin, a β₃-adrenergic receptor agonist substance, can be utilized as a pharmaceutical agent for treating or preventing obesity in human. As described in the following examples, the present inventors indeed confirmed that dried lotus leaf extracts which contain quercetin has effects of improving lipid metabolism in humans and mice. The dried lotus leaf extract containing quercetin is useful as a pharmaceutical agent/food for treating or preventing obesity not only in humans but also in mammals including rodents such as mice. When using a dried quercetin-containing lotus leaf extract for treating or preventing obesity, the amount of intake is not limited as long as it is within a safe and effective range. If examples of the amount of intake are to be provided, it would be 0.01 g/day to 100 g/day, and preferably 0.1 g/day to 10 g/day for humans. If the amount is expressed in terms of the amount of quercetin containing the aglycone and glycoside forms, the amount of intake is within a range of, for example, 0.88 mg/day to 8.82 g/day, and preferably 8.82 mg/day to 0.88 g/day. The *in vitro* effects of improving fat metabolism can be evaluated, for example, by adding a test substance to adipocytes and measuring the amount of glycerol (i.e., a product of lipid degradation). The amount of glycerol can be determined at the end by carrying out absorbance measurements, after degradation with glycerol kinase and such. If the amount of intracellular glycerol increases because of the test substance, the test substance can be determined as having a lipid metabolism-improving effect. *The in vivo* effect can be evaluated by feeding a high-fat diet and the test substance to some of the test animals, and a high fat diet without the test substance to another group; breeding the two groups of animals for a specified period of time under identical conditions; and then comparing the amount of visceral fat between the animal groups. If the amount of visceral fat in the test compound-fed group is less than that of the high-fat diet-only group, the test substance is evaluated to have a lipid metabolism-improving effect.

When formulating a quercetin-comprising β₃-adrenergic receptor agonist substance into a pharmaceutical product, the dosage form can be selected depending on factors such as therapeutic purpose and route of administration. Examples include tablets, pills, powders, liquids, suspensions, emulsions, granules, capsules, injections, suppositories, elixirs, syrups, infusions or decoctions, and tinctures. Diluents or excipients such as fillers, expanders, binding agents, moisturizers, disintegrators, surfactants, or lubricants can be used for formulation as necessary. Furthermore, coloring agents, preservatives, perfumes, flavors, sweeteners, and other pharmaceutical products can be included in the pharmaceutical formulation.

The type of food that contains a quercetin-comprising β₃-adrenergic receptor agonist substance includes tea, health tea, health foods, foods for specified health uses, dietary supplements, and enteral nutrition foods. Compounds that are acceptable in terms of food hygiene, such as stabilizers, preservatives, coloring agents, flavors, or vitamins, are suitably added to and mixed with a quercetin-comprising β₃-adrenergic receptor agonist substance to produce foods in a form of tablet, particle, granule, powder, capsule, liquid, cream, or drink. When producing pharmaceutical products and foods, plants containing quercetin such as lotus can be suitably used as a raw material containing a quercetin-comprising β₃-adrenergic receptor agonist substance. The methods for preparing a quercetin-comprising β₃-adrenergic receptor agonist substance from plants are described above. An example of the preparation methods is described in detail in the following examples.

All prior art references cited herein are incorporated by reference into this description.

### [Examples]

Hereinbelow, the present invention will be specifically described with reference to Examples, but it is not to be construed as being limited thereto.

### [Example 1] Production of lotus leaf extracts

Ten liters of water was added to 1 kg of dried lotus leaves. The mixture was adjusted to pH 6.0 and left to stand at room temperature for 30 minutes. Extraction was then carried out by boiling this mixture under reduced pressure at 90°C for one hour. Filtrate 1 was separated from the residue. Ten equivalents of water was then added to the residue. Then, extraction was carried out again by boiling this mixture under reduced pressure at 90°C for one hour, whereupon filtrate 2 and residue were obtained by separation. Filtrate 1 and filtrate 2 were combined, concentrated by heating under reduced pressure to a specific gravity of 1.1, and then dried using a spray drier to obtain 100 g of dried powder.

### [Example 2] Identification and purification of quercetin and quercetin glycoside

The lotus leaf extract was was analyzed by LC/MS. Capcell Pak C18 UG120 φ2.0x 150 mm (Shiseido) was used for the column. For the mobile phase, solution A (5% aqueous acetonitrile solution containing 1 % acetic acid) and solution B (acetonitrile containing 1% acetic acid) were selected. Elution was carried out in 30 minutes with a linear concentration gradient from solution A to solution B. Conditions for the reversed-phase HPLC were column temperature: 40°C; injection amount: 5 µL; and elution rate: 200 µL/min. Ionization was performed by ESI (Negative). Commercially available quercetin (quercetin dehydrate; Wako Pure Chemicals), quercitrin (quercetin-3-rhamnoside; Tokyo Chemical Industry), and isoquercitrin (quercetin-3-glucoside; EXTRASYNTHESE) were used as control samples and analyzed in the same way.

Since the quercetin peak (RT: 16.4 min, m/z: 301) matched the RT and m/z of the control sample, the presence of quercetin (molecular weight: 302) in the lotus leaf extract was confirmed. Similarly, quercitrin (molecular weight: 448; RT: 13.8 min, m/z: 447), and isoquercitrin (molecular weight: 464; RT: 12.9 min; m/z: 463) were found to be present in the lotus leaf extract. The RT: 13.2 min, m/z: 477 peak was presumed to be that of quercetin-3-glucuronide (Q3GA; molecular weight: 478).

Purification of Q3GA from the lotus leaf extract and its structural determination were carried out as follows.

First, 1 g of the lotus leaf extract was dissolved in 500 mL of ultrapure water (MilliQ water), and the solution was adjusted to pH 3 using 6 N hydrochloric acid. The solution was extracted 3 times with 500 mL of ethyl acetate, and the extract was then dried over anhydrous magnesium sulfate. This ethyl acetate layer was concentrated under reduced pressure to obtain an acidic fraction (147 mg, 14.7% yield). Next, the acidic fraction was purified through 30 rounds of HPLC with 5 mg of sample at a time. The conditions for HPLC purification were as follows. Capcell Pak C18 UG120 φ2.0x 250 mm (Shiseido) was used as a column for purification. Column temperature was 40°C and the injection amount was 200 µL. 50% aqueous acetonitrile solution containing 1% acetic acid was used as the mobile phase, elution was performed at a given elution rate (5 mL/min), and detection was carried out at 360 nm. These conditions were used to obtain a Q3GA-containing fraction (63.1 mg, 42.1% yield). This fraction was further purified using Sephadex LH-20 (φ12x 350 mm, MeOH) to obtain a compound (13.5 mg) which is presumed to be Q3GA.

Next, the obtained compound was analyzed by NMR. ¹H-NMR, ¹³C-NMR, DEPT, H-HCOSY, HMQC, and HMBC measurements were taken, and since a comparison to literature values (J. Agric. Food Chem., 46, pp.4898-4903 (1998)) showed that the measured values match the literature values for Q3GA, this confirmed the presence of Q3GA in the lotus leaf extract.

When concentrations of the quercetin and quercetin glycoside present in the above-prepared dried lotus leaf extract were calculated, the amount of the total aglycone (quercetin) and glycoside was 88.2 mg/g.

### [Example 3] Production of recombinant cells expressing human β₃-adrenergic receptor

Human β₃-adrenergic receptor cDNA was synthesized by the PCR method using a human small intestine-derived cDNA library (TaKaRa) as template, and the following synthetic oligo DNAs as primers.
5' oligo primer: ccgctagccaccatggctccgtggcctcacgagaag (SEQ ID NO: 1)
3' oligo primer: ccgaattctacccgtcgagccgttggcaaag (SEQ ID NO: 2)

After desalting with Sephacryl S-300 and removing the unreacted primers, the PCR-synthesized cDNAs were digested at restriction enzyme sites *Nhe*I and *Eco*RI, which were pre-inserted at the ends of the primers during primer design, and then by using a ligation kit (TaKaRa), ligation with an animal expression vector pTracer-EF A (Invitrogen), which had been digested with *Spe*I and *Eco*RI restriction enzymes, was carried out. The ligated DNAs were precipitated using ethanol, and then suspended in a suitable amount of 10% aqueous glycerol solution. This DNA solution was then used to transform the *E. coli* DH5α strain by electroporation. The manipulated cells were plated onto an LB agar plate (ampicillin-containing medium), and cultured overnight at 37°C to obtain colonies of transformants. The cDNA nucleotide sequences of human β₃-adrenergic receptor in ten transformants were confirmed using an Applied Biosystems automatic sequencer, and recombinant plasmids in which the correct human β₃-adrenergic receptor cDNA had been inserted into the animal expression vector were selected.

The human β₃-adrenergic receptor-expressing recombinant plasmids were extracted from *E*. *coli* cells by alkaline lysis (Sambrook & Russell, Molecular Cloning, 3rd Edition), and then purified. The purified recombinant plasmids were transfected into CHO-K1 cells, which are Chinese hamster ovary cells. Transfection was carried out by the lipofectin method using the TransIT-LT1 Reagent (TaKaRa). TransIT-LT1 mixed with 10 µg of the recombinant plasmid was added to cells that had grown to a cell density of 20 to 30% in a 10-cm dish, and then the plasmid was incorporated into the cells. After transfection, the cells were cultured for 3 days in Dulbecco's modified Eagle medium (hereinafter, abbreviated as DMEM; Sigma) supplemented with 10% fetal calf serum (hereinafter, abbreviated as FCS) at 37°C in a 5% CO₂ atmosphere. The medium was replaced with Zeocin (500 µg/mL; Invitrogen) supplemented DMEM, and the cells were cultured under similar conditions. Cells that had grown in the presence of Zeocin and formed colonies were detached with trypsin, and then grown in DMEM containing Zeocin and 10% FCS.

The obtained recombinant cells were cultured in a 96-well microplate until the cell density reaches 100%. After the medium was removed, the cells were washed once with Dulbecco's modified phosphate buffer solution (hereinafter, abbreviated as PBS; TaKaRa), and then 100 µL of an assay buffer [DMEM, 10% FCS, 20 mM HEPES (pH 7.2), 0.1 mM isobutylmethylxanthine] containing 10 µM isoproterenol was added. As a control, the assay buffer was used without addition of isoproterenol. After incubation at 37°C for 20 minutes, the cells were washed once with PBS, and then the amount of intracellular cAMP were quantified using a cAMP EIA Kit (Amersham Bioscience). The recombinant cells, in which the intracellular cAMP level had greatly increased because of the isoproterenol added as a β-adrenergic receptor agonist, were selected. The selected recombinant cells were further cultured. After the cells were detached with trypsin, they were diluted with a culture medium and dispensed into a 96-well microplate at 1 cell per well. These cells were further cultured and their responsiveness to isoproterenol was confirmed with a similar procedure. Recombinant cells that showed good responsiveness were purified, and ultimately a single human β₃-adrenergic receptor-expressing recombinant, 6H-4d3, was obtained.

### [Example 4] Measurement of human β₃-adrenergic receptor agonist activity

The human β₃-adrenergic receptor-expressing recombinant 6H4-2d3 was cultured in a 96-well microplate in DMEM containing 10% FCS and 500 µg/mL Zeocin at 37°C in a 5% CO₂ atmosphere. After culturing the cells for 2 to 3 days until they have grown to a density of about 100%, the medium was removed. The cells were washed once with PBS, and an assay buffer [DMEM, 10% FCS, 20 mM HEPES (pH 7.2), 0.1 mM isobutylmethylxanthine] supplemented with the test substance to be measured was added at 100 µL/well. After incubation at 37°C for 10 minutes, the cells were washed once with PBS, and then their intracellular cAMP levels were quantified using a cAMP EIA Kit (Amersham Bioscience). As a negative control, 6H4-2d3 cells treated with the assay buffer alone, or the assay buffer plus an equal amount of a solution used to dissolve the test substance, were used. As a positive control, 6H4-2d3 cells treated with isoproterenol were used. Furthermore, to confirm that the increase of intracellular cAMP occurred as a result of β₃-adrenergic receptor-specific reactions, CHO-K1 cells, which are parent cells of the 6H4-2d3 cells, were treated in exactly the same way and the change in intracellular cAMP was confirmed. The same test substance was measured three times and the mean and standard deviation of the measurements were used.

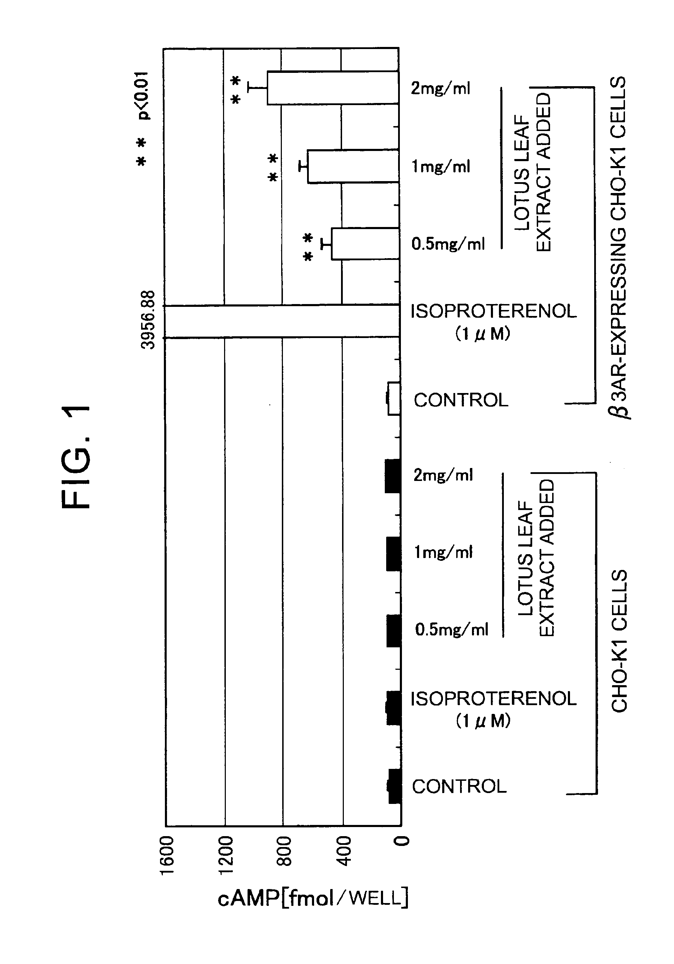

To investigate the β₃-Adrenergic receptor agonist activity of the dried lotus leaf extract, the above-mentioned measurements were performed using the dried lotus leaf extract as a test substance. More specifically, the dried lotus leaf extract was added to a test medium at concentrations of 0.5 mg/mL, 1 mg/mL, and 2 mg/mL, and the response was examined in CHO-K1 cells expressing the human β₃-adrenergic receptor and in CHO-K1 cells that do not express the receptor. 1 µM isoproterenol was used for the positive control. As a result, only in the CHO-K1 cells expressing the human β₃-adrenergic receptor, a significant accumulation of cAMP due to addition of the dried lotus leaf extract was observed. Therefore, it was clear that the extract has β₃-adrenergic receptor agonist activity. The results are shown in Fig. 1.

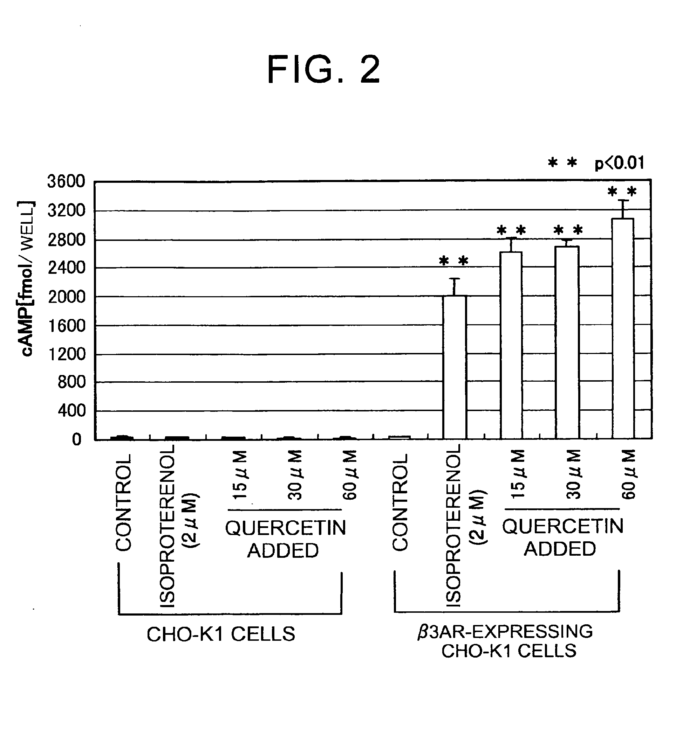

To investigate also the human β₃-adrenergic receptor agonist activity of quercetin, similar measurements were performed using quercetin as a test substance. Quercetin, a major ingredient of dried lotus leaf extract, was added to a test medium at concentrations of 15 µM, 30 µM, and 60 µM, and the response was examined in CHO-K1 cells expressing the human β3-adrenergic receptor and in CHO-K1 cells that do not express the receptor. 2 µM isoproterenol was used for the positive control. As a result, only in the CHO-K1 cells that express the human β₃-adrenergic receptor, a significant accumulation of cAMP due to quercetin addition was observed. Thus, it was clear that quercetin has β₃-adrenergic receptor agonist activity. The results are shown in Fig. 2.

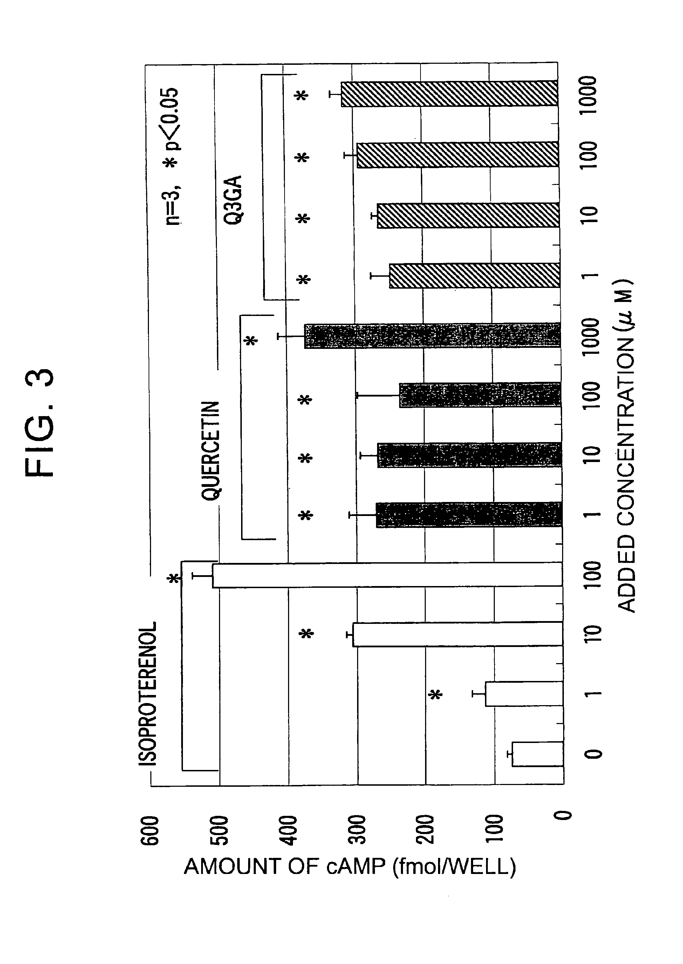

Furthermore, the β-adrenergic receptor agonist activity of Q3GA was also examined. Q3GA was added to a test medium at concentrations of 1 µM, 10 µM, 100 µM, and 1000 µM, and the response was examined in CHO-K1 cells expressing the human β₃-adrenergic receptor. For comparison, the same procedure was performed using quercetin. Isoproterenol was used as the positive control. As a result, addition of Q3GA (i.e., a glycoside) was found to have a β₃-adrenergic receptor agonist activity similar to that of quercetin, which is an aglycone. The results are shown in Fig. 3.

### [Example 5] Measurement of lipolytic effect using 3T3-L1 cells

3T3-L1 cells which are adipocyte precursor cells derived from mice (purchased from Human Science Research Resource Bank) were added into a 96-well plate at 1x10⁴ cells/well, and then cultured in Dulbecco's modified Eagle medium (D-MEM, GIBCO) supplemented with 10% fetal calf serum (FCS) at 37°C in a 5% CO₂ atmosphere. To induce differentiation to adipocytes, right before the cells reaches confluency, the medium was exchanged with 10% FCS-containing D-MEM supplemented with 0.5 mM 3-isobutyl-1-methylxanthine, 2x 10⁻⁷ M dexamethasone, and 0.8 µM insulin. Two days later, the medium was exchanged with 10% FCS-containing D-MEM supplemented only with 0.8 µM insulin. The culture medium was exchanged every 2 to 3 days and culturing was continued until the cells differentiated into adipocytes. The medium was exchanged against 10% FCS-containing D-MEM, and the cells were cultured for 2 days. The culture medium of the cells cultured in the 96-well plate were removed by aspiration, 10% FCS-containing D-MEM containing the test substance was added at 100 µL/well, and after incubation for 3 days, 80 µL of the culture medium was collected from each well, and the amount of glycerol in the culture medium was determined using "F-Kit Glycerol" (Boehringer Mannheim). A significant difference test for comparing the mean values was carried out using Student's t-test, and p<0.05 was defined as significant.

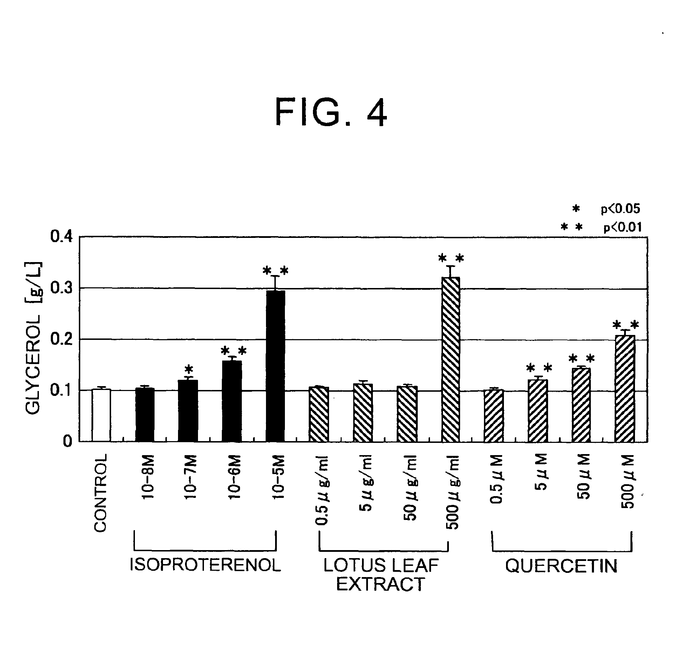

To investigate the lipolytic effects of the dried lotus leaf extract and quercetin in adipocytes, the above-mentioned measurements were performed. To a test medium, 0.5 to 500 µg/mL of dried lotus leaf extract, 0.5 to 500 µM of quercetin, and 10⁻⁸ M to 10⁻⁵ M of isoproterenol as a positive control were added individually, and the amount of glycerol released into the medium due to degradation of the fat stored in 3T3-L1 adipocytes was measured. Lipolysis was found to be significantly promoted as a result of adding 500 µg/mL of dried lotus leaf extract, or adding quercetin in the range of 5µM to 500 µM. The results are shown in Fig. 4.

### [Example 6]

After a four-day preliminary breeding of 5-week old female Wistar rats, the rats were divided into groups such that the average body weight was approximately the same for each group. Each group consisted of 8 animals, and 4 groups were formed: normal diet group, high-fat diet group, high-fat diet + 0.01 g/g lotus leaf extract (0.01 g of lotus leaf extract per 1 g of high-fat diet, denoted similarly hereinafter) intake group, and high-fat diet + 0.05 g/g lotus leaf extract intake group. In the high-fat diet groups, starch, sucrose, lard oil (10%), corn oil (10%), and cholesterol (1%) were add to the feed. After breeding each group for 2 weeks, the animals were dissected. The animals were fasted for 6 hours before the dissection began. Fat in the abdominal cavity (intraperitoneal fat) was collected and weighed. The amount of intraperitoneal fat for every 100 g body weight was 2.2340 ± 0.6427 g for the normal diet group, 3.9071 ±1.2562 g for the high-fat diet group, 3.5564 ± 0.8805 g for the high-fat diet + 0.01 g/g lotus leaf extract intake group, and 2.7745 ± 0.9099 g for the high-fat diet + 0.05 g/g lotus leaf extract intake group. All of these measurements are expressed as mean ± S.D..

### [Example 7] Hypoglycemic effects of lotus leaf extract on KK-A^{y} mice, a mouse model of type 2 diabetes

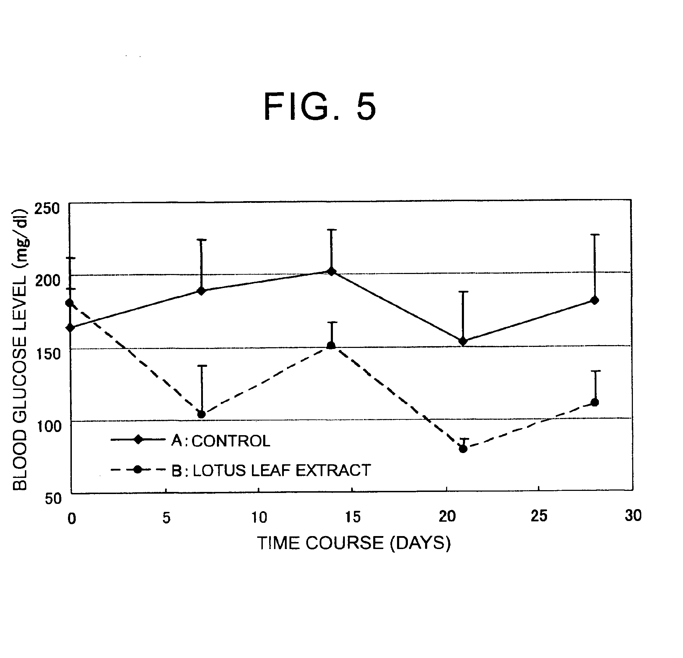

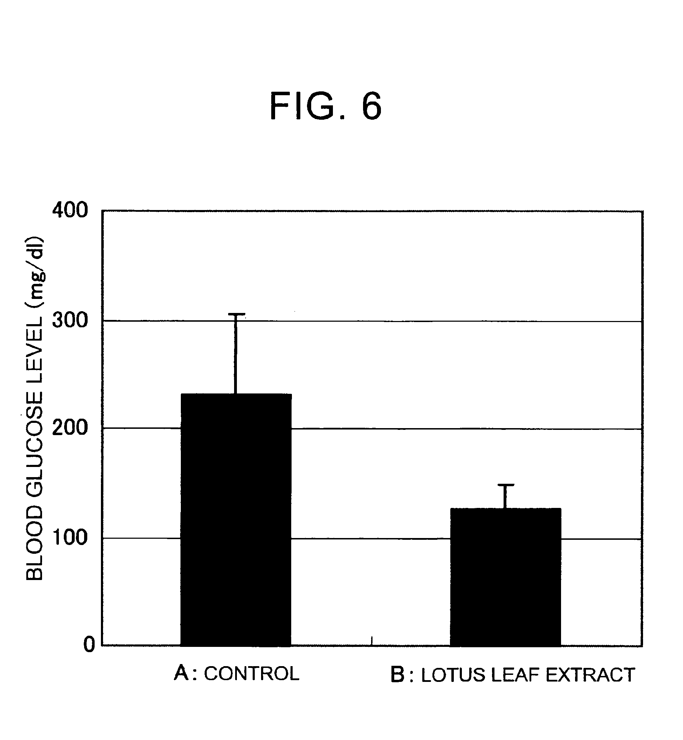

After a three-day preliminary breeding of 4-week old male KK-A^{y} mice, a mouse model of type 2 diabetes, the mice were divided according to weight into two groups of 10 animals each. After dividing the groups, blood glucose levels were measured. The solvent control group was given tap water, and the group supplied with lotus leaf extract-containing water was given an aqueous lotus leaf extract solution produced by dissolving the dried lotus leaf extract at 10 mg/mL. The animals were allowed to drink freely and eat (CRF-1) freely. Blood glucose levels were measured every week. The blood glucose level measurements were taken after 6 hours of fasting. Non-fasting blood glucose level on Day 25 of the administration was also measured. The results are shown in Figs. 5 and 6.

### [Example 8] Analysis of the effect of dried lotus leaf extracts in improving obesity in high-fat diet-loaded obese mice

### 8-(1) Experimental materials and methods

The effects of administering dried lotus leaf extracts as test substances to high-fat-diet-loaded mice were examined. Six-week old female ICR mice were obtained from CLEA Japan, and were quarantined and conditioned for 14 days under rearing conditions the same as the test conditions. On the day of administration forty animals, which showed satisfactory weight gain during quarantine and conditioning with no abnormalities in their general condition, were used in the tests. The mice were grouped by stratified random sampling based on weight. The four test groups were: Group A (basic feed); Group B (high-fat diet); Group C (high-fat diet containing 2% dried lotus leaf extract); and Group D (high-fat diet containing 5% dried lotus leaf extract). Each group consisted of ten animals. The mice were raised in two cages per group, with five mice of the same group housed in each cage. Powdered feed CE-2 (CLEA Japan) was used as the basic feed. The other feeds (the high-fat diet and high-fat diets containing dried lotus leaf extract) are described in Table 1. The values in the table show the amount of each ingredient to mix when preparing a total of one kilogram of feed mixture.

**[Table 1] Preparation of the high-fat diets and the weighed amount of test substance**

| | High-fat diet | High-fat diet containing 2% dried lotus leaf extract | High-fat diet tract containing 5% dried lotus leaf extract |
|---|---|---|---|
| CE-2 | 460g | 460g | 460g |
| Beef tallow | 400g | 400g | 400g |
| Granulated sugar | 90g | 90g | 90g |
| Corn starch | 50g | 30g | 0g |
| Dried lotus leaf extract | 0g | 20g | 50g |

In all groups, the feed was placed in a powder feeder, and the animals were free-fed. The water supply was tap water placed in a water supply bottle, which was free-fed through a nozzle. The test substance was continuously administered to the mice for ten weeks under the above-mentioned conditions. The general condition of the mice was observed, their body weight determined, the amount consumed was measured, and the mice were examined by autopsy, yielding the results below. The data shown below for body weight, feed intake, and organ weight are expressed as a mean ± S.D. Student's t-tests or Dunnett's multiple comparison tests were used to determine significant differences between the group on basic feed and the group on a high-fat diet; and between the group on a high-fat diet and the groups on the test substance.

### 8-(2) Observation of general condition

The general condition of every cage was observed once a day. Abnormalities were not observed in the general condition of any individual for the duration of the examination period.

### 8-(3) Body weight

An electronic top-loading balance was used to measure body weight immediately before administration of the test substance, and once a week after administration. The results for each group are individually shown in Table 2.

**[Table 2]**

| Body weight (g) of mice during high-fat diet tolerance test | | | | | | | |
|---|---|---|---|---|---|---|---|
| Group | | Before test | After starting test (weeks) | | | | |
| | | | 1 | 2 | 3 | 4 | 5 |
| A: | Basic feed | 30.4 ±1.3 | 31.7 ±1.9 | 32.6 ±1.9 | 33.6 ±1.7 | 33.8 ±1.8 | 34.8 ±2.1 |
| B: | High-fat diet | 30.5 ±1.3 | 32.7 ±1.4 | 34.7 ±1.2** | 35.6 ± 2.1 * | 37.5±2.6** | 39.2±3.4** |
| C: | High-fat diet + 2% dried lotus leaf extract | 30.5±1.3 | 32.2 ±2.2 | 34.9 ±2.7 | 36.1±3.0 | 36.9 ±3.0 | 37.7 ± 3.2 |
| D: | High-fat diet + 5% dried lotus leaf extract | 30.5±1.3 | 31.0 ±1.4 | 33.4 ±1.7 | 34.5 ±2.3 | 35.8 ±2.7 | 35.5 ±19# |

| Group | | | After starting test (weeks) | | | | |
|---|---|---|---|---|---|---|---|
| | | | 6 | 7 | 8 | 9 | 10 |
| A: | Basic feed | | 35.2 ± 2.4 | 35.6 ± 2.9 | 36.6 ± 2.8 | 38.9 ± 3.5 | 39.3 ±3.0 |
| B: | High-fat diet | | 40.2 ±2.8** | 41.5 ±4.3** | 43.3 ± 4.2** | 45.9 ±5.4** | 46.2 ±5.6** |
| C: | High-fat diet + 2% dried lotus leaf extract | | 38.6 ±4.0 | 38.7 ±4.0 | 40.8 ±3.8 | 41.4 ±5.3 | 43.0 ± 5.7 |
| D: | High-fat diet + 5% dried lotus leaf extract | | 38.0 ± 2.9 | 38.6 ± 1.5## | 38.1 ±3.0## | 39.3 ±3.3## | 42.2 ±3.2 |

| | | | | | | | |
|---|---|---|---|---|---|---|---|
| Each value represents mean ± S.D. (n=10) | | | | | | | |
| *. P<0.05 : | | | | | | | |
| **, P<0.01 (basic feed group vs. high-fat diet group: Student's t-test) #. P<0.05: ##, P<0.01 (high-fat diet group vs. high-fat diet + dried lotus leaf extract group: Dunnett's test) | | | | | | | |

Group A (basic feed) and Group B (high-fat diet) were compared to examine the effect of a high-fat diet. The results showed that the body weight of mice in both groups increased steadily after starting the test, but that the body weight of mice in Group B increased more than in Group A. Significant differences were observed between the body weights of the two groups from two weeks after starting the test, up until completion of the test. At the end of the test, there was an average difference of 6.9 g between the two groups (Group A, 39.3 ± 3.0 g; Group B, 46.2 ± 5.6 g; p<0.01).

Group B, Group C (high-fat diet containing 2% dried lotus leaf extract), and Group D (high-fat diet containing 5% dried lotus leaf extract) were compared to analyze the effect of the dried lotus leaf extract. After starting the test the body weights of all groups increased steadily, but the increase in body weight in Group C and Group D tended to be less than in Group B. Significant differences between Group D and Group B in particular were confirmed at 5, 7, 8, and 9 weeks after starting the test. Accordingly, dried lotus leaf extract was confirmed to have the effect of suppressing increases in body weight.

### 8-(4) Feed intake

Feed intake was measured twice a week, that is, every three or four days after test substance administration. The weight of the feed including the feeder was measured for each cage using an electronic top-loading balance, and intake was calculated by subtracting the weight of the remaining feed from the weight of the feed provided.

The average daily feed intake per animal, calculated for each cage, is shown in Table 3. For each cage, n=5; and for each group, n=10.

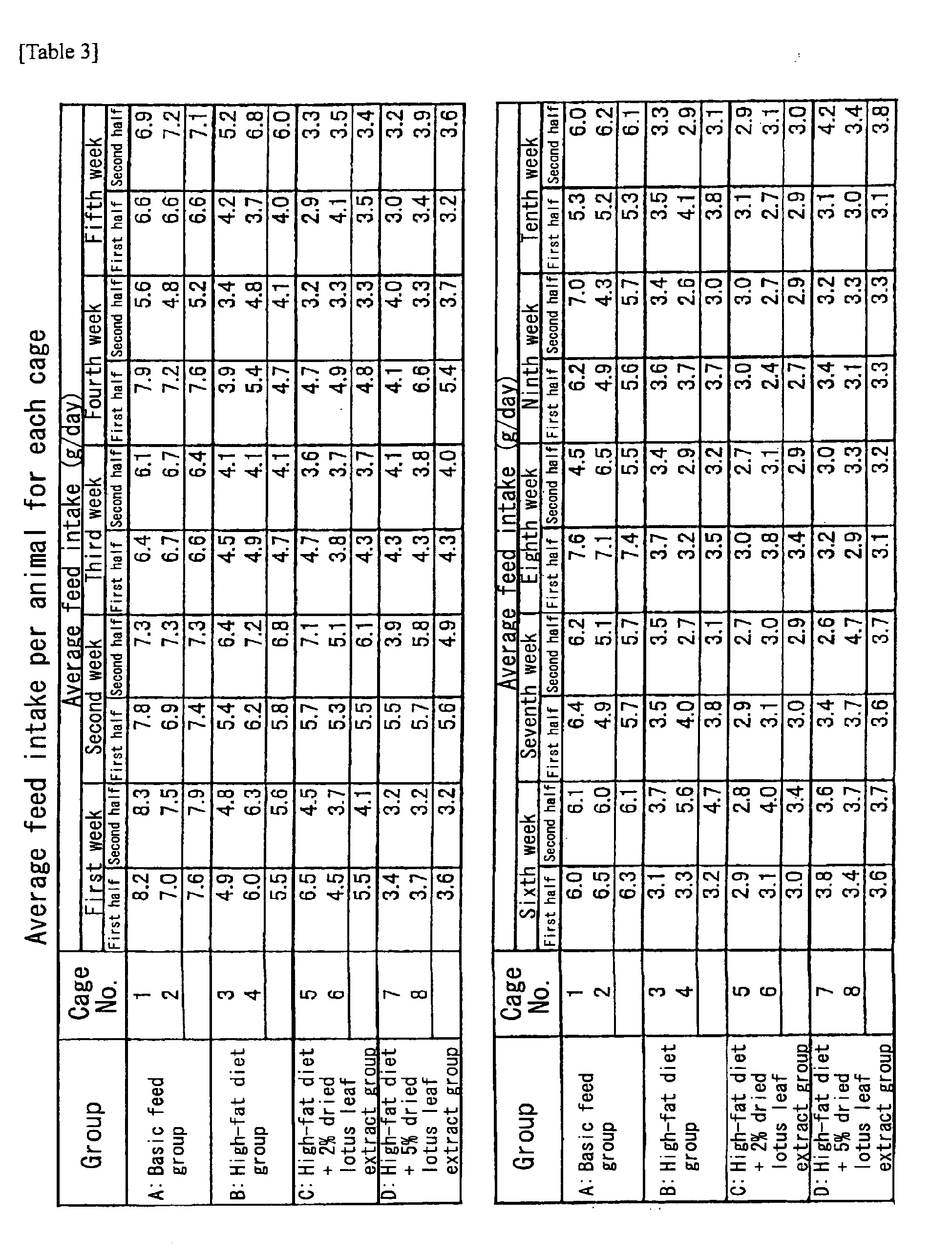

The average daily feed intake per animal was 5.2 to 7.9 g/day in Group A, 3.0 to 6.8 g/day in Group B, 2.7 to 6.1 g/day in Group C, and 3.1 to 5.6 g/day in Group D. The intake for Group A was clearly greater than in the other groups. Although feed intake tended to be greater in the first half of the test (around the second week) and less in the latter half of the test (sixth week and beyond), there was no particular change in each of Groups B to D.

### 8-(5) Organ weight

At the end of the observation period (ten weeks after starting the test), the mice were autopsied and their organ weights (wet weights of the liver, kidneys, and fat) were measured. The results are shown in Table 4. In each group, n=10.

**[Table 4]**

| Mouse organ weight on completion of the high-fat diet tolerance test | | | | | |
|---|---|---|---|---|---|
| | Group | Number | Total liver weight (g) | Kidney weight (g) | Fat weight (g) |
| A: | Basic feed | 10 | 1.9332 ± 0.2201 | 0.4164 ± 0.0387 | 1.5620 ± 0.6598 |
| B: | High-fat diet | 10 | 2.0428 ± 0.3107 | 0.3680 ±0.0368* | 3.3871 ± 1.3395** |
| C: | High-fat diet + 2% dried lotus leaf extract | 10 | 1.9430 ± 0.2213 | 0.3463 ± 0.0312 | 2.6391 ± 1.0912 |
| D: | High-fat diet + 5% dried lotus leaf extract | 10 | 2.0869 ± 0.2408 | 0.3934 ±0.0358 | 1.7292 ± 0.5215## |

| | | | | | |
|---|---|---|---|---|---|
| Each value represents mean ± S.D. | | | | | |
| *, P<0. 05; **, P<0.01 (basic feed group vs. high-fat diet group: Student' s t-test or Aspin-Walch's t-test) #, P<0. 05 ; ##, P<0.01 (high-fat diet group vs. high-fat diet + dried lotus leaf extract group: Dunnett's test) | | | | | |

When Groups A and B were compared to analyze the effect of the high-fat diet, no significant difference was observed for the liver weight. However, in Group B the kidneys were significantly lighter, and the fat was significantly heavier.

When Groups B, C, and D were compared to analyze the effect of the dried lotus leaf extract, the liver and kidney weights did not show significant differences, but the fat weight was low in both Groups C and D. Significant differences were observed between Group D and Group B in particular, confirming the effect of the dried lotus leaf extract in reducing fat.

### 8-(6) Summary

As described above, although the group taking 2% dried lotus leaf extract did display a trend towards suppressed increase in body weight, the effect was not significant. On the other hand, significant suppression of body weight increase was observed in the group taking 5% dried lotus leaf extract. However, since there was some difference in feed intake, further examination was required to determine whether the weight loss was due to the level of feed intake or to the effect of the dried lotus leaf extract. Accordingly, the amount of feed necessary to increase body weight by 1 g was calculated for each group, using total feed intake during the test and the amount of weight increase at completion of the test. This amount was 19.1 g for the group on a high-fat diet, 20.2 g for the group taking 2% dried lotus leaf extract (high-fat diet + 2% dried lotus leaf extract), and 22.7 g for the group taking 5% dried lotus leaf extract (high-fat diet + 5% dried lotus leaf extract). This finding confirmed a dose-dependent increase. Therefore, it can be concluded that the suppression of weight increase observed in this Example was a result of the dried lotus leaf extract. The weight of fat at the time of autopsy tended to be reduced in the group taking 2% dried lotus leaf extract, and was significantly reduced in the group taking 5% dried lotus leaf extract, and these results support the above conclusion.

### [Example 9] Effect of dried lotus leaf extract on carbohydrate metabolism and lipid metabolism in humans

### 9-(1) Experimental materials and methods

An objective of this Example was to analyze the effect of the dried lotus leaf extract on carbohydrate metabolism and lipid metabolism in humans. To carry out analyses with the above aim, human subjects were limited to those who satisfied a given set of conditions. A subject in this Example must satisfy the following conditions:
1) They must be a so-called borderline-type subject. Specifically, the subject's fasting blood glucose level must be 110 to 126 mg/dl, or their blood glucose level two hours after taking a 75 g glucose tolerance test (OGTT) must be 140 to 200 mg/dl. Individuals whose fasting blood glucose level is 126 mg/dl or more, or whose blood glucose level at two hours after 75 g OGTT is 200 mg/dl or more, were defined as diabetes-type subjects and were therefore excluded from the subjects of this Example.
2) They must have a Body Mass Index (BMI) of 22 or more. BMI is also called physique index and is a value determined by dividing a subject's body weight (kg) by the square of their height (m). BMI = body weight (kg) / height (m)²
3) They must not have received medication for diabetes.
4) They must not have a severe liver, kidney or cardiovascular disorder, or a food allergy.
5) They must be 40 to 58 years old if male, and 40 to 55 years old if female, and their daily life must be similar to that of a healthy individual.
6) Their daily intake of tea must be 2 L or less.

Questionnaires and such were given to those individuals who satisfied the above six conditions, and subjects were selected. The questionnaire and interview asked the following: A) daily calorie intake; B) food preference; and C) medical history, family history, work and living environment, exercise habits, and smoking and drinking habits.

95 individuals who matched the subject conditions were selected as subjects. The selected subjects were divided into three groups such that there was no difference between the groups in terms of age, sex, and fasting blood glucose level. Two of the groups took dried lotus leaf extract (Group T and Group R) and the other took a placebo (Group S). As the control group, individuals in Group S consumed a basic tea (200 mL/bottle) that did not comprise the lotus leaf extract. As the test substance-taking groups, individuals in Group T consumed a tea comprising 0.5 g/200 mL of dried lotus leaf extract powder, and individuals in Group R consumed a tea comprising 1.0 g/200 mL of dried lotus leaf extract powder. The constitution and average age of each group are shown in Table 5. Group S (placebo): n=30; Group T (1 g of dried lotus leaf extract/day): n=34; and Group R (2 g of dried lotus leaf extract/day): n=31.

**[Table 5] Group constitution and average age in human test groups**

| Group constitution | | Average age ± S.D. (number of subjects) |
|---|---|---|
| S | (placebo) | 48.3 ± 4.7(n=30) |
| T | (1 g of dried lotus leaf extract/day) | 50.6±5.0(n=34) |
| R | (2 g of dried lotus leaf extract/day) | 48.9±4.6(n=31) |

The test was carried out using a double-blind test with the placebo group as a control. The subjects were observed for two weeks prior to intake of the test food, and then consumed two bottles of the above-mentioned tea per day (200 mL x 2/day), one bottle in the morning and another in the afternoon, for 12 weeks. Physical measurements and sugar tolerance tests were carried out on the subjects to analyze the effect of the dried lotus leaf extract.

### 9-(2) Physical measurements

Physical measurements were taken for each subject before intake, and six and 12 weeks after intake. The measured characteristics were height, weight, waist circumference, BMI, hip circumference, percent body fat, and level of visceral fat. Percent body fat and level of visceral fat were measured using an Omron Body Composition Monitor HBF-352. A visceral fat level of 10 corresponds to visceral fat area of 100 cm². The results are shown in Tables 6 and 7. For both Tables 6 and 7, Group S (placebo): n=30; Group T (1 g of dried lotus leaf extract/day): n=34; and Group R (2 g of dried lotus leaf extract/day): n=31.

**[Table 6]**

| Human tests (Physique Index 1) | | | | | | Mean ± s. D. |
|---|---|---|---|---|---|---|
| | Group | Pre-test value | Value at six weeks | Value at twelve weeks | Change (at six weeks) | Change (at twelve weeks) |
| Body weight (kg) | S | 71.7±10.0 | 71.0±9.9 | 70.5±10.1 | -0.7±0.9 | -1.2±1.5 |
| | T | 71.5±11.3 | 70.2±11.0 | 69.2±10.9 | -1.3±1.1 | -2.3±1.6^{#} |
| | R | 71.5±9.7 | 69.7±9.9 | 68.9±10.1 | -1.8±1.1* | -2.7±1.4* |
| BMI | S | 25.6±2.4 | 25.3±2.4 | 25.1 ±2.4 - | -0.3±0.3 | - 0.5±0.5 |
| | T | 25.7±2.8 | 25.3±2.8 | 24.9±2.7 | -0.4±0.4 | -0.8±0.5^{#} |
| | R | 25.6±2.5 | 24.9±2.6 | 24.6±2.6 | -0.7±0.5* | -1.0±0.6* |
| Percent body fat (%) | S | 28.6±5.2 | 28.1±5.5 | 27.0±5.7 | -0.6±1.1 | -1.6±1.3 |
| | T | 29.3±4.7 | 27.7±4.5 | 26.7±4.4 | -1.5±1.3^{#} | -2.6±1.9^{#} |
| | R | 28.8±4.6 | 27.0±4.5 | 25.7±5.1 | -1.7±1.0 * | -3.0±1.6* |

| | | | | | | |
|---|---|---|---|---|---|---|
| [S vs T]p<0.05 : # [S vs R] p<0.05: * (Dunnett's test) | | | | | | |

**[Table 7]**

| Human tests (Physique Index 2) | | | | | | Mean±s.D. |
|---|---|---|---|---|---|---|
| | Group | Pre-test value | Value at six weeks | Value at twelve weeks | Change (at six weeks) | Change (at twelve weeks) |
| Visceral fat level | S | 10.4±3.3 | 10.1±3.3 | 9.8±3.3 | -0.3±0.7 | -0.6±0.7 |
| | T | 11.4±4.0 | 10.9±4.0 | 10.3±3.9 | -0.5±0.7 | -1.1±1.2# |
| | R | 11.3±3.9 | 10.3±4.0 | 9.8±3.9 | -0.9±0.6* | -1.4±0.7* |
| Waist ference (cm) | S | 86.9±7.3 | 86.5±7.3 | 86.3±7.2 | -0.4±0.6 | -0.6±1.1 |
| | T | 87.1±8.6 | 85.9±8.5 | 84.9±8.2 | -1.2±1.4* | -2.2±1.7^{#} |
| | R | 87.2±8.0 | 85.3±7.9 | 84.8±7.9 | -1.9±1.2* | -2.4±1.7* |
| Hip circumference (cm) | S | 97.9±6.3 | 97.5±6.1 | 97.3±6.0 | -0.3±1.1 | - 0.5±1.4 |
| | T | 96.7±6.9 | 96.4±6.7 | 95.7±6.6 | -0.3±0.8 | -1.0±2.0^{#} |
| | R | 97.9±6.4 | 96.5±5.7 | 96.3±5.8 | -1.4±1.8 | -1.6±2.1* |

| | | | | | | |
|---|---|---|---|---|---|---|
| [S vs T] p<0.05 : # [S vs R] p<0.05:* (Dunnett's test) | | | | | | |

Compared to subjects in the control group, subjects taking 2 g/day of dried lotus leaf extract for six and 12 weeks showed significantly lowered body weight, BMI, percent body fat, visceral fat level, waist circumference, and hip circumference, showing the effect of dried lotus leaf extract in reducing such values. Taking 1 g/day of dried lotus leaf extract for six weeks also significantly lowered body weight and percent body fat compared to the control group. Intake for 12 weeks significantly lowered body weight, BMI, percent body fat, visceral fat level, and waist circumference compared to the control group.

### 9-(3) Glucose tolerance tests

Glucose tolerance tests (75 g glucose/body) were conducted twice for each subject: once before intake and once 12 weeks after intake. The subjects were not allowed to eat or drink after 9 p.m. the night before blood collection. On the day of testing, blood was first collected under fasting conditions and taken to be blood before glucose loading. Next, a 75 g glucose load was given, and blood was collected over time, that is, at 30, 60, and 90 minutes after loading. The test was completed by 11 a.m. Blood glucose level in the plasma was measured using a Hitachi automatic analyzer 7075.

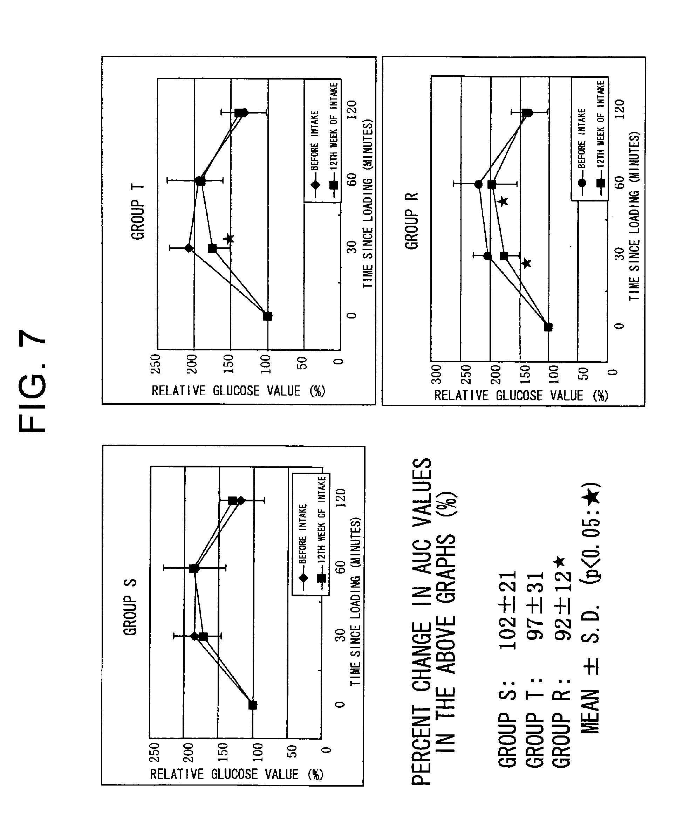

The results are shown in Fig. 7. In Fig. 7, the blood glucose level at each time point is expressed as a relative glucose level (%) in comparison to the blood glucose level before the glucose load is given. Furthermore, based on the relative glucose levels at each time point, the area under the curve (AUC) was calculated for 0 to 120 minutes. Group S (placebo): n=30; Group T (1 g of dried lotus leaf extract/day): n=34; and Group R (2 g of dried lotus leaf extract/day): n=31. Intake of dried lotus leaf extract for 12 weeks was shown to suppress blood glucose increase at 30 minutes and 60 minutes after glucose loading.

### 9-(4) Summary

As described above, as for the animal tests, dried lotus leaf extract was found to have the effect of reducing body fat in humans. The reduction in body fat due to a daily intake of 2 g of dried lotus leaf extract for six weeks was reflected as a reduction in weight in humans. A similar effect was also confirmed for an intake of 1 g/day for 12 weeks. Such effects and the results of glucose tolerance tests suggest that the lotus leaf extracts have the effect of reducing body fat as well as reducing insulin resistance.

### Industrial Applicability

The present invention made it possible to provide quercetin as a β₃-adrenergic receptor agonist substance. In particular, the substances of the present invention can be used as new options for obesity improvement and diabetes treatment.

## Claims

1. A β₃-adrenergic receptor agonist substance comprising quercetin.

2. The substance of claim 1, wherein the quercetin is derived from a plant.

3. The substance of claim 2, wherein the plant is a *Nelumbonaceae* plant.

4. A pharmaceutical agent for treating or preventing diabetes, wherein the agent comprises the substance of any one of claims 1 to 3.

5. A pharmaceutical agent for treating or preventing obesity, wherein the agent comprises the substance of any one of claims 1 to 3, and has an effect of improving lipid metabolism.

6. A food for treating or preventing diabetes, wherein the food comprises the substance of any one of claims 1 to 3.

7. A food for treating or preventing obesity, wherein the food comprises the substance of any one of claims 1 to 3.

8. A β₃-adrenergic receptor agonist substance which comprises a lotus preparation comprising quercetin.


SEQUENCE LISTING

<110>  MEIJI DAIRIES CORPORATION

<120>  PLANT-ORIGIN BETA 3-ADRENOCEPTOR AGONIST AND USE OF THE SAME

<130>  M1-A0302P

<150>  JP 2003-374836
<151>  2003-11-04

<160>  2

<170>  PatentIn version 3.1

<210>  1
<211>  36
<212>  DNA
<213>  Artificial

<220>
<223>  An artificially synthesized primer sequence

<400>  1
ccgctagcca ccatggctcc gtggcctcac gagaag                                36

<210>  2
<211>  31
<212>  DNA
<213>  Artificial

<220>
<223>  An artificially synthesized primer sequence

<400>  2
ccgaattcta cccgtcgagc cgttggcaaa g                                     31